(19) Europäisches Patentamt / European Patent Office / Office européen des brevets

(11) **EP 2 465 570 B1**

(12) **EUROPÄISCHE PATENTSCHRIFT**

(45) Veröffentlichungstag und Bekanntmachung des Hinweises auf die Patenterteilung:
**30.03.2016  Patentblatt 2016/13**

(51) Int Cl.:
***A61N 1/05*** *(2006.01)*     *A61N 1/08* *(2006.01)*

(21) Anmeldenummer: **11191737.3**

(22) Anmeldetag: **02.12.2011**

(54) **Implantierbares Gerät**

Implantable device

Appareil implantable

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.12.2010  US 424071 P**

(43) Veröffentlichungstag der Anmeldung:
**20.06.2012  Patentblatt 2012/25**

(73) Patentinhaber: **BIOTRONIK SE & Co. KG**
**12359 Berlin (DE)**

(72) Erfinder:
 • **Dörr, Thomas**
 **12437 Berlin (DE)**

 • **Weiss, Ingo**
 **12435 Berlin (DE)**

(74) Vertreter: **Galander, Marcus et al**
**Biotronik SE & Co. KG**
**Corporate Intellectual Properties**
**Woermannkehre 1**
**12359 Berlin (DE)**

(56) Entgegenhaltungen:
**US-A1- 2004 199 071     US-A1- 2007 288 058**
**US-A1- 2009 281 592     US-A1- 2010 016 936**

## Beschreibung

Die Erfindung betrifft ein permanent oder temporär implantierbares Gerät mit einem langgestreckten elektrischen Leiter.

Solche Geräte, beispielsweise Elektrodenleitungen für die Elektrostimulation, haben den Nachteil, dass sich ihr elektrischer Leiter in einem Kernspintomografen erwärmen kann, weil die im Kernspintomografen herrschenden wechselnden Magnetfelder in dem elektrischen Leiter nicht unbeachtliche elektrische Ströme induzieren. Auch können solche induzierten Ströme über Elektrodenpole der Elektrodenleitung an umliegendes Gewebe abgegeben werden und so beispielsweise zu unerwünschten Gewebeerwärmungen führen. Deshalb können Herzschrittmacherpatienten heutzutage in der Regel nicht oder nur eingeschränkt in einem Kernspintomografen untersucht werden.

An implantierbaren Herzschrittmachern oder Defibrillatoren sind nämlich typischerweise wenigstens eine Stimulationselektrodenleitung angeschlossen, die an ihrem proximalen, zum Anschluss an den Herzschrittmacher oder Defibrillator vorgesehenen Ende einen standardisierten elektrischen Anschluss aufweist und an ihrem distalen, zur Platzierung im Herzen vorgesehenen Ende einen oder mehrere Elektrodenpole aufweist. Ein solcher Elektrodenpol dient zur Abgabe elektrischer Impulse an das Gewebe (Myokard) des Herzens oder zum Abfühlen elektrischer Felder, um im Rahmen des sogenannten Sensings eine Aktivität eines Herzens abfühlen zu können. Zu diesen Zwecken bilden Elektrodenpole typischerweise elektrisch leitende Oberflächenabschnitte einer Elektrodenleitung. Elektrodenpole sind typischerweise als Ringelektrode in Form eines Rings um die Elektrodenleitung oder in Form einer Spitzen- oder Tip-Elektrode am distalen Ende der Elektrodenleitung vorgesehen. Die Elektrodenpole sind über eine oder mehrere elektrische Leiter mit Kontakten des elektrischen Anschlusses der Elektrodenleitung an deren proximalem Ende elektrisch leitend verbunden. Somit verlaufen zwischen den Kontakten des elektrischen Anschlusses die Elektrodenleitungen an deren proximalen Ende und den Elektrodenpolen am distalen Ende der Elektrodenleitung ein oder mehrere elektrische Leiter, die einen oder mehrere der Elektrodenpole mit einem oder mehreren der Kontakte elektrisch verbinden. Diese elektrischen Leiter können einerseits zur Übertragung von Stimulationsimpulsen zu den Elektrodenpolen und andererseits zur Übertragung mittels der Elektrodenpole aufgenommener elektrischer Signale zum proximalen Ende der Elektrodenleitung genutzt werden und werden im Verlauf der weiteren Beschreibung auch jeweils als Funktionsleitung bezeichnet. Solche Funktionsleitungen sind für die Funktionen der jeweiligen Elektrodenleitung erforderliche elektrische Leiter und als solche der Gefahr ausgesetzt, dass in ihnen durch äußere Wechselmagnetfelder elektrische Ströme induziert werden, die beispielsweise zu einer unerwünschten Erwärmung der Funktionsleitungen oder der mit ihr verbundenen Elektrodenpole führen können oder die zur Abgabe entsprechender Ströme über die Elektrodenpole an umgebendes Gewebe und damit zu einer Erwärmung des umgebenden Gewebes führen können.

Der Erfindung liegt die Aufgabe zugrunde, ein Gerät zu schaffen, welches das zuvor beschriebene Problem löst. Ein Gerät gemäß dem ersten Teil des Anspruchs 1 ist aus US 2010/0016936 A1 bekannt (siehe Abb. 22).

Erfindungsgemäß wird diese Aufgabe durch ein Gerät gemäß Anspruch 1 gelöst, welches wenigstens zwei langgestreckte elektrische Funktionsleitern für die Übertragung von Therapiesignalen oder Diagnosesignalen oder beidem, und einen mit einem der Funktionsleiter verbundenen Elektrodenpol aufweist, über den elektrischer Strom an im Benutzungsfall umgebendes Körpergewebe abgegeben oder mit dem elektrische Potentiale in im Benutzungsfall umgebenden Gewebe abgefühlt werden können oder beides. Die beiden elektrischen Funktionsleiter für definierte Resonanzfrequenzen sind derart induktiv gekoppelt, dass RF-Energie eines ersten Funktionsleiters auf einen zweiten Funktionsleiter abgeleitet wird und die Energie über diesen Funktionsleiter und einen mit diesem Funktionsleiter verbundenen Elektrodenpol an im Benutzungsfall umliegendes Gewebe abgeben wird. Auf diese Weise wird es möglich, im Einsatzfall induzierte RF-Energie gezielt auf einen zur Abgabe dieser Energie geeigneten Elektrodenpol abzuleiten.

Gemäß einer bevorzugten Ausführungsvariante ist das medizinische Gerät ein bi- oder mehrpolarer, temporär anwendbarer Katheter oder eine dauerimplantierbare Elektrodenleitung oder ein anderes langgestrecktes elektrisch leitfähiges Implantat mit einer teilweisen Isolation, so dass an definierten Elektrodenflächen eine lokale Erwärmung durch im MRT induzierte Ströme zu erwarten ist, wobei mittels einer induktiven Kopplung für definierte Resonanzfrequenzen die RF-Energie einer ersten Zuleitung als erstem Funktionsleiter auf eine zweite oder weitere Zuleitung als jeweiligen zweiten Funktionsleiter abgeleitet wird, der die Energie dann über einen Elektrodenpol an das umliegende Gewebe abgibt.

Der mit dem jeweiligen zweiten Funktionsleiter verbunden Elektrodenpol wird vorzugsweise von wenigstens einer Ring-Elektrode gebildet. Diese Ringelektrode kann eine funktionale Ringelektrode sein, die auch zur Abgabe von Stimulationsimpulsen oder zur Erfassung von Potentialen dient. Alternativ kann die Ringelektrode auch lediglich zum Ableiten induzierter Energie vorgesehen sein, also keine weitere Funktion haben, so dass sie im Folgenden als nichtfunktional bezeichnet wird. Im letztgenannten Sinn ist eine Ausführungsvariante bevorzugt, bei der der zweite Funktionsleiter wenigstens mit einer zusätzlichen Ringelektrode als speziell zur Ableitung induzierter RF-Energie vorgesehenem Elektrodenpol elektrisch verbunden ist.

Vorzugsweise ist das medizinische Gerät eine Stimulationselektrodenleitung für den Anschluss an einen dauerhaft implantierbaren Stimulator, wie beispielsweise einen Herzschrittmacher oder Defibrillator, um den Trägern solcher Implantate Untersuchungen in einem Magnet-Resonanz-Tomographen (MRT) möglich zu machen.

Zur induktiven Kopplung der beiden Funktionsleiter ist erfindungsgemäß zwischen den ersten und den zweiten Funktionsleiter ein Transformator geschaltet. Zusätzlich ist vorzugsweise ein mit einer Wicklung des Transformators parallel oder in Reihe geschalteter Kondensator zur Abstimmung der Resonanzfrequenz der induktiven Kopplungsschaltung aus Transformator und Kondensator vorgesehen. Vorzugsweise ist der Kondensator mit der Sekundärwicklung des Transformators verbunden, die ihrerseits wiederum mit dem zweiten Funktionsleiter verbunden ist.

Transformator und ein ggf. vorhandener Kondensator sind vorzugsweise so aufeinander abgestimmt, dass sich eine gewünschte Resonanzfrequenz unter Berücksichtigung einer im Benutzungsfall zu erwartenden parasitären Kapazität umgebenden Körpergewebes und umgebender Körperflüssigkeit ergibt.

Besonders bevorzugt ist es, wenn der Transformator kernlos ist. Alternativ kann der Transformator einen Kern aus ferromagnetischem Kernmaterial besitzen. In diesem fall besteht der Kern vorzugsweise aus ferromagnetischem Kernmaterial, dessen Sättigung erst bei größerer als der zu erwartenden MRT-Magnetfeldstärke einsetzt.

Die Erfindung soll nun anhand von Ausführungsbeispielen mit Bezug auf die Figuren näher erläutert werden. Die Figuren zeigen Folgendes:

Fig. 1    zeigt als implantierbare medizinische Geräte einen implantierbaren Herzstimulator 10 und eine daran angeschlossene implantierbare Elektrodenleitung 20.

Fig. 2    zeigt ein Beispiel eines Temperaturverlaufs an der Elektrodenspitze.

Fig. 3a    und 3b zeigen Beispiele eines zur induktiven Kopplung zweier Funktionsleiter geeigneten MRI-Resonators.

Fig. 4    zeigt einen MRI-Resonator Variante 2.

Fig. 5    zeigt einen MRI-Resonator mit zwei Ableitungen.

Fig. 6    zeigt einen MRI-Resonator mit zwei Ableitungen Variante 2.

Fig. 7    zeigt einen MRT-Resonator mit mehreren nicht-therapeutischen Ringen zur Energieableitung.

Fig. 8    zeigt einen MRT Resonator mit einer nichttherapeutischen Ringelektrode.

Der implantierbare Herzstimulator 10 kann ein Herzschrittmacher oder ein Kardioverter/Defibrillator (ICD) sein. Im dargestellten Ausführungsbeispiel ist der Herzstimulator 10 ein ventrikulärer Herzschrittmacher und Defibrillator. Andere bekannte Herzstimulatoren sind Zweikammerherzschrittmacher zur Stimulation des rechten Atriums und des rechten Ventrikels oder biventrikuläre Herzschrittmacher, die zusätzlich zum rechten Ventrikel auch den linken Ventrikel stimulieren können.

Derartige Stimulatoren besitzen typischerweise ein Gehäuse 12, das im Regelfall aus Metall besteht und somit elektrisch leitend ist und als großflächiger Elektrodenpol dienen kann. An der Außenseite des Gehäuses 12 ist typischerweise ein Anschlussgehäuse 14 befestigt, das auch als Header bezeichnet wird. Ein derartiger Header weist typischerweise Kontaktbuchsen zur Aufnahme von Steckkontakten auf. Die Kontaktbuchsen besitzen elektrische Kontakte 16, die über entsprechende Leiter mit einer in dem Gehäuse 12 des Herzstimulators 10 angeordneten Elektronik verbunden sind.

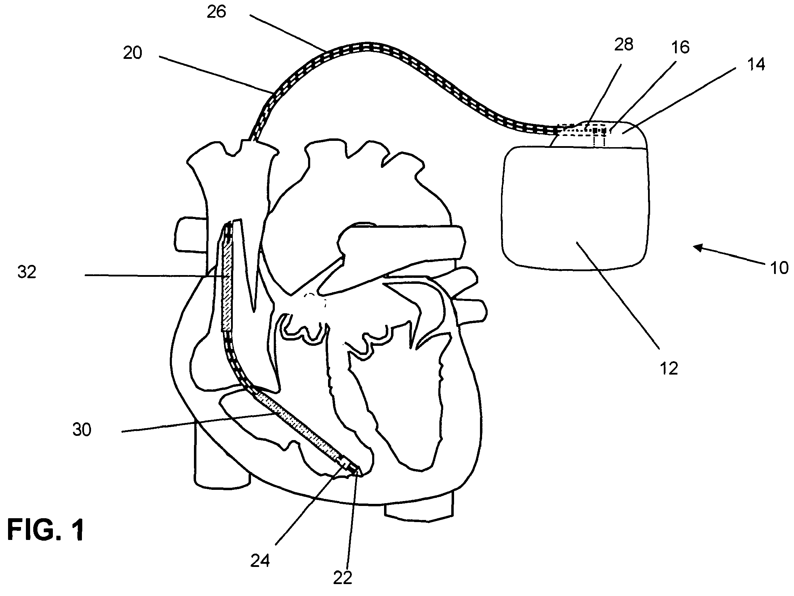

Die Elektrodenleitung 20 stellt im Sinne dieser Erfindung ebenfalls ein implantierbares medizinisches Gerät dar. Am distalen Ende der Elektrodenleitung 20 sind in an sich bekannter Manier Elektrodenpole in Form einer Spitzen- oder Tip-Elektrode 22 und einer in deren Nähe angeordneten Ringelektrode 24 angeordnet. Die Elektrodenpole 22 und 24 sind so ausgebildet, dass sie je nach Funktion eines Herzstimulators, an den die Elektrodenleitung 20 angeschlossen ist, zum Abfühlen elektrischer Potentiale des Herzgewebes (Myokards) dienen oder zur Abgabe elektrischer Signale, beispielsweise zur Abgabe von Stimulationsimpulsen an das sie umgebende Herzgewebe, ausgebildet sind. Fig. 1 zeigt, wie sich die Elektrodenpole, also die Tip-Elektrode 22 und die Ringelektrode 24, im Anwendungsfall die Elektrodenleitung 20, im Apex eines rechten Ventrikels eines Herzens befinden.

Sowohl die Tip-Elektrode 22 als auch die Ringelektrode 24 sind über jeweils wenigstens einen elektrischen Leiter 26 mit einem Steckkontakt 28 am proximalen Ende der Elektrodenleitung 20 elektrisch verbunden. Der Steckkontakt 28 besitzt elektrische Kontakte, die mit den elektrischen Kontakten 16 der Kontaktbuchse im Anschlussgehäuse 14 des implantierbaren Herzstimulators korrespondieren. Die elektrischen Leiter 26 in der Elektrodenleitung 20 können als annähernd gestreckte Seilzugleiter oder als helixförmig gewendelte Leiter ausgebildet sein. Solche Leiter, die funktionale Elektrodenpole mit elektrischen Kontakten des Steckkontaktes am proximalen Ende der Elektrodenleitung 20 elektrisch leitend verbinden werden im Rahmen dieses Textes als Funktionsleiter bezeichnet, da sie beispielsweise der Therapie dienende elektrische Signale von Steckkontakt zum jeweiligen Elektrodenpol übertragen oder abgefühlte elektrische Potentiale repräsentierende Signale vom jeweiligen Elektrodenpol zum Steckkontakt führen und somit der elementaren Funktion des medizinischen Gerätes dienen.

Die elektrischen Leiter 26, die die Elektrodenpole 22 bzw. 24 mit den elektrischen Kontakten des Steckers 28 der Elektrodenleitung 20 verbinden, sind über den größten Teil ihrer Länge von einer isolierenden Hülle umgeben, so dass ein elektrischer Kontakt zum Gewebe des Herzens gezielt über die Elektrodenpole zustande kommt.

Neben den Elektrodenpolen 22 und 24, die typischerweise der (in diesem Fall ventrikulären) Stimulation des Herzgewebes dienen, weist die Elektrodenleitung 20 auch noch zwei großflächigere Elektrodenpole 30 und 32 auf, die als Defibrillationselektroden dienen und von wenigstens einem blank liegendem helixartig gewendelten Draht gebildet sind.

Es sei darauf hingewiesen, dass die Erfindung im Rahmen dieses Ausführungsbeispiels anhand eines rechtsventrikulären Herzschrittmachers und Defibrillators erläutert wird. Als medizinisches Gerät im Sinne der Erfindung kann grundsätzlich aber beispielsweise auch eine Ablationselektrodenleitung dienen, die im Anwendungsfall ebenfalls bis ins Herz eines Patienten hineinragt und die von einem außerhalb des Patienten angeordneten Gerät gesteuert wird und hierzu mit diesem verbunden ist.

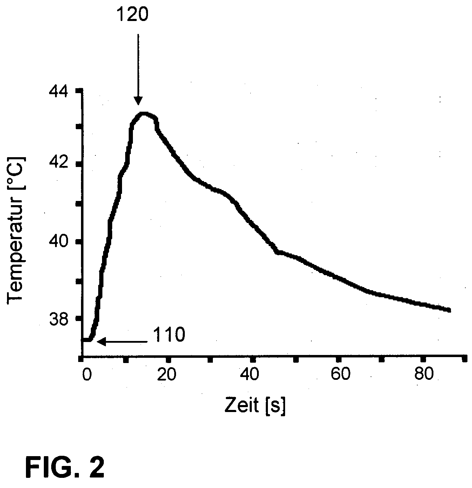

In Figur 2 ist ein typischer Temperaturverlauf 100 einer konventionellen Schrittmacher/ICD-Elektrode im Magnet-Resonanz-Tomographen (MRT) dargestellt. Mit dem Einschalten des hochfrequenten Wechselfeldes im Tomographen zum Zeitpunkt 110 steigt die Temperatur rasch an, wobei die Steilheit des Anstieges und die maximal erreichbare Temperatur stark von der Elektrodenlage bezogen auf die hochfrequenten Wechselfelder des MRT abhängig ist. Wird das hochfrequente Wechselfeld abgeschaltet (zum Zeitpunkt 120), dann kühlt sich die Elektrodenspitze aufgrund ihrer vergleichsweise geringen Wärmekapazität verhältnismäßig schnell wieder ab.

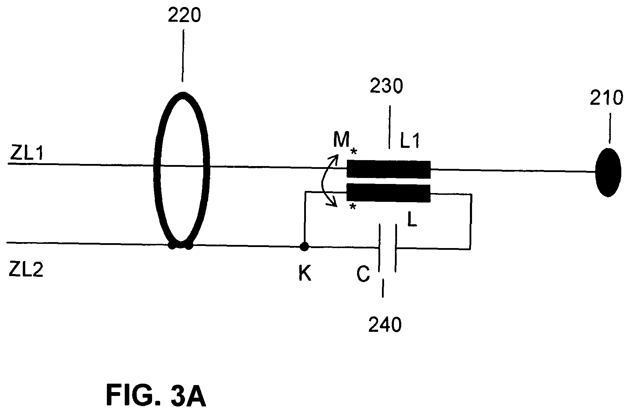

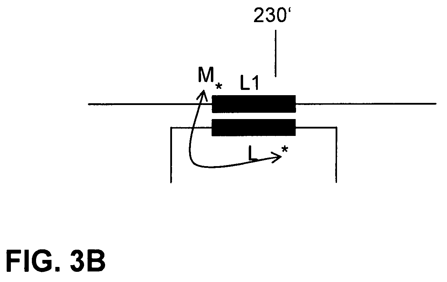

Die Figuren 3 bis 8 zeigen in schematisch vereinfachter Darstellung jeweils zwei Funktionsleiter am distalen Ende einer Elektrodenleitung. Die Funktionsleiter sind jeweils mit ZL1 (für erste Elektrodenzuleitung) und ZL2 (für zweite Elektrodenzuleitung) bezeichnet. Der jeweils erste Zuleiter ZL1 ist mit einer jeweiligen Tip-Elektrode 210, 310, 410, 510, 610 bzw. 710 als Elektrodenpol verbunden, während der jeweilige zweite Funktionsleiter ZL2 mit einer Ringelektrode 220, 320, 420, 520 bzw. 720 als Elektrodenpol verbunden ist. Weitere typische Bestandteile von Elektrodenleitungen wie eine isolierende Hülle oder Anschlusskontakte am jeweils proximalen Ende sind der Einfachheit halber weggelassen.

In Figur 3 ist die erfindungsgemäße Ableitung der MRT-induzierten Ströme auf die Ringelektrode 220 am zweiten Funktionsleiter ZL2 dargestellt. Das Prinzip ist es, mit dem gezeigten Schwingkreis im Resonanzfall Tip-Elektrode 210 und Ring-Elektrode 220 kurzzuschließen. Hierzu ist ein Transformator 230 zwischen den ersten und den zweiten Funktionsleiter ZL1 und ZL2 geschaltet. Ein Kondensator 240, der zur Sekundärwicklung L des Transformators 230 parallel geschaltet ist, dient der Abstimmung der Resonanzfrequenz. Diese Anordnung erlaubt kleine Bauelementegrößen, insbesondere Spulen mit sehr kleiner Induktivität und ist damit gut mit dem Elektrodendesign vereinbar.

Die Resonanzfrequenz berechnet sich nach:

$$f_0 = \frac{1}{2\pi\sqrt{LC}}$$

Für ein 1,5T MRT ist somit bei einer Kapazität C = 1pF "nur" eine Induktivität von etwa 6,5$\mu$H im Resonanzkreis nötig. Eine solche Anordnung ist ggf. hinter einer Ringelektrode unterzubringen.

In einer anderen bevorzugten Realisierung wird C>10pF gewählt, da sonst die kernlose (wegen Sättigung im statischen Magnetfeld des MRI) Realisierung zu viele Windungen/ große Geometrie erfordert.

In der in Figur 3 dargestellten Ausführungsvariante ist der Transformator kernlos. In einer anderen bevorzugten Realisierung wird ein Kern verwendet der jedoch erst bei Feldstärken größer als die des anvisierten MRT sind (z.B. Materialien, die erst bei ca. 1.7 T sättigen). Damit können Elektroden mit sehr effektivem Kerntrafo für 1T und 1.5 T MRT Einsatz gebaut werden.

Der Kontaktpunkt K ist in Figur 3a beispielhaft an der Zuleitung ZL2, also dem zweiten Funktionsleiter vorgesehen. Gegenstand der Erfindung sind auch alle Realisierungsvarianten wo ein Kontaktpunkt K am ersten Funktionsleiter ZL1 kontaktiert ist. In diesem Fall ergibt sich ein Reihenschwingkreis LC der Sekundärwicklung L des Transformators 230 und des Kondensators 240.

Alle Realisierungen sind auch Gegenstand der Erfindung wenn der Übertrager, mit dem Transformator 230', umgekehrt gekoppelt ist, wie dies in Fig. 3b dargestellt.

In Figur 3 bezeichnet:

| | |
|---|---|
| 210: | Tip-Elektrode |
| 220: | Ringelektrode |
| 230: | Trafo (mit oder ohne Kern) |
| 240: | Kondensator |
| ZL1: | Zuleitung Tip-Elektrode |
| ZL2: | Zuleitung Ring-Elektrode |

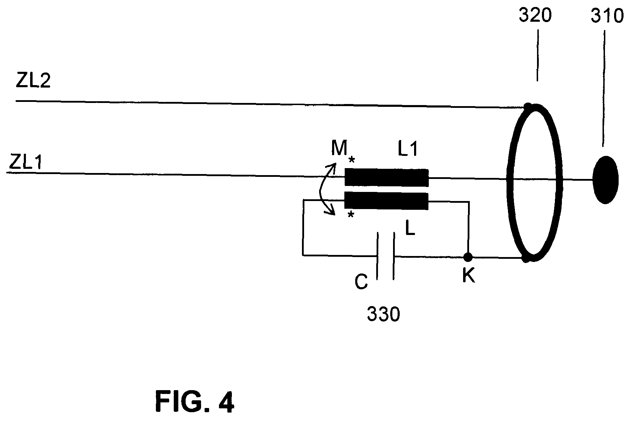

In Figur 4 ist eine alternative Ausführungsform dargestellt. Hier ist der Resonator 330 mit dem Transformator und dem Kondensator proximal der Ringelektrode 320 angebracht. Diese Ausführung bietet den konstruktiven Vorteil, dass keine Versteifung der Elektrode im Bereich der Elektrodenspitze erforderlich ist.

In Figur 4 bezeichnet:

310:    Tip-Elektrode
320:    Ringelektrode
330:    Trafo und Kondensator (Resonator)
ZL1:    Zuleitung Tip-Elektrode
ZL2:    Zuleitung Ring-Elektrode

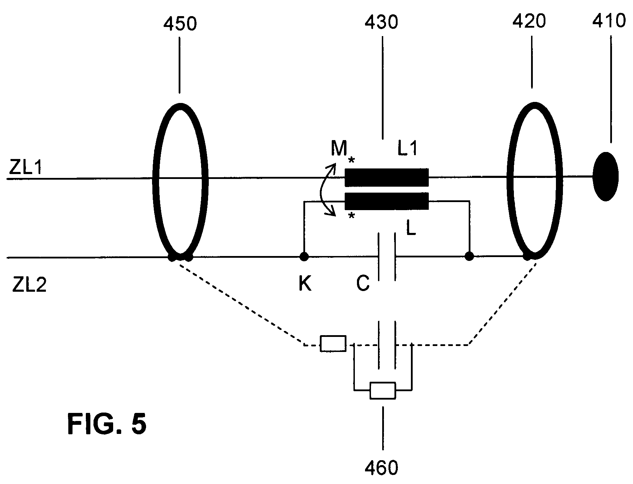

In der Abbildung 5 ist eine erweiterte Ausführungsform dargestellt, bei der eine zusätzliche, nicht-funktionale Ringelektrode 450 vorgesehen ist, die mit dem zweiten Funktionsleiter ZL2 verbunden ist. Hier erfolgt die Ableitung der MRT induzierten RF-Energie zusätzlich auf eine nichtfunktionale Ringelektrode 450.

Bei dieser Anordnung ist bei der Dimensionierung immer auch das parasitäre Körper RC-Netzwerk 460 zu berücksichtigen bzw. zu nutzen. Das parasitäre Körper RC-Netzwerk 460 ergibt sich im Einsatzfall - als nach Implantation - aus den elektrischen Eigenschaften der umgebenden Körperflüssigkeiten und des umgebenden Körpergewebes.

Vorteil dieser Variante ist die Möglichkeit, größere Energien ableiten zu können und gleichzeitig die Dimensionierung der funktionalen Ringelektrode 420 nicht auf die Anforderungen der Wärmeableitung optimieren zu müssen.

In Figur 5 bezeichnet:

410:    Tip-Elektrode
420:    Funktionale Ringelektrode
430:    Trafo und Kondensator (Resonator)
450:    zusätzliche Ringelektrode zur Wärmeableitung
460:    parasitäres Körpernetzwerk
ZL1:    Zuleitung Tip-Elektrode
ZL2:    Zuleitung Ring-Elektrode

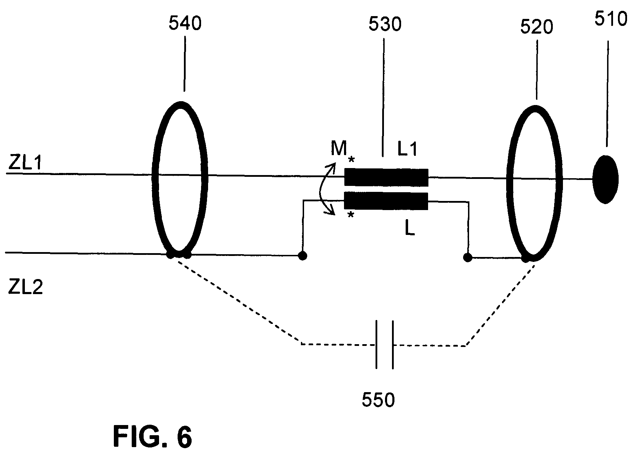

In Figur 6 ist eine gegenüber Figur 5 vereinfachte Ausführungsform dargestellt, die ebenfalls eine zusätzliche, nicht-funktionale zweite Ringelektrode 540 am zweiten Funktionsleiter ZL2 aufweist. Hier erfolgt die Ableitung der MRT induzierten ebenfalls RF-Energie zusätzlich auf die nicht-funktionale Ringelektrode 540.

Bei dieser Anordnung entfällt jedoch der Kondensator im Resonatorkreis 530. Die für die Resonanz benötigte Kapazität wird durch die im Einsatzfall zu erwartende, parasitäre Körperkapazität 550 in der Dimensionierung ersetzt.

In Figur 6 bezeichnet:

510:    Tip-Elektrode
520:    Funktionale Ringelektrode
530:    Trafo
540:    zusätzliche Ringelektrode zur Wärmeableitung
550:    parasitäre Körperkapazität
ZL1:    Zuleitung Tip-Elektrode
ZL2:    Zuleitung Ring-Elektrode

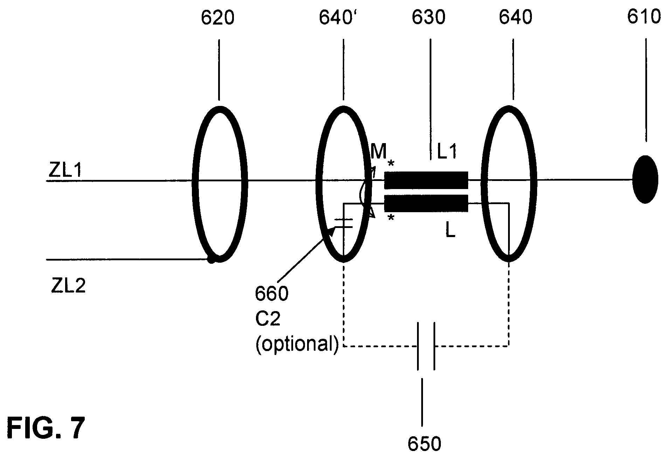

In der Figur 7 ist eine Ausführungsform mit mehreren nichtfunktionalen Ringelektroden 640, 640' zur Wärmeableitung dargestellt. Das Grundprinzip entspricht hier der Ausführungsvariante gemäß Figur 6, bietet jedoch den Vorteil, dass größere Energiemengen abgeleitet werden können.

Die funktionale Ringelektrode 620 wird darüber hinaus nicht von der zusätzlichen Beschaltung beeinflusst. Die Ausführungsvariante gemäß Figur 7 kommt somit auch grundsätzlich ohne zweiten Funktionsleiter aus, so dass die Leitungsabschnitte zwischen der Sekundärwicklung L des Transformators 530 und der jeweiligen Ringelektrode 640 bzw. 640' als zweite Funktionsleiter im Sinne der Erfindung wirken.

In Figur 7 bezeichnet:

610:    Tip-Elektrode
620:    Funktionale Ringelektrode
630:    Trafo
640, 640':    zusätzliche Ringelektroden zur Wärmeableitung
650:    parasitäre Körperkapazität
660:    optionaler Kondensator zur Anpassung an den Resonanzfall
ZL1:    Zuleitung Tip-Elektrode
ZL2:    Zuleitung Ring-Elektrode

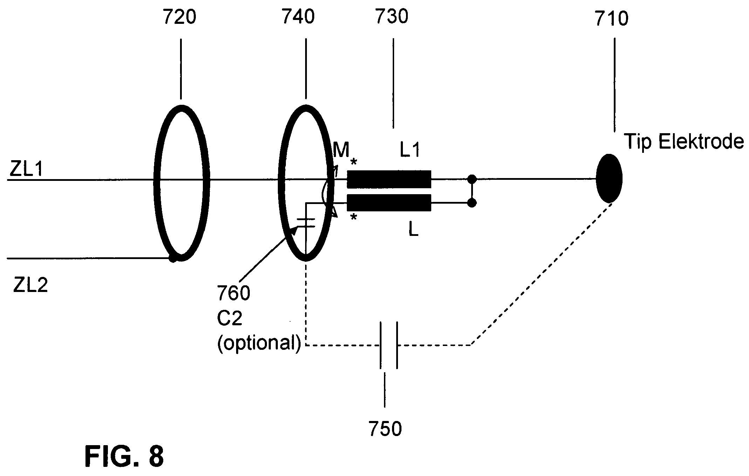

Bei der in Figur 8 dargestellten Ausführungsvariante ist das Prinzip der Ausführungsvariante gemäß Figur 7 vereinfacht. Hier erfolgt die Energieableitung an eine nichtfunktionale Ringelektrode 740. Die funktionale Ringelektrode 720 wird durch die zusätzliche Beschaltung jedoch nicht beeinflusst.

In Figur 8 bezeichnet:

710:    Tip-Elektrode
720:    Funktionale Ringelektrode
730:    Trafo
740:    zusätzliche Ringelektrode zur Wärmeableitung
750:    parasitäre Körperkapazität
760:    optionaler Kondensator zur Anpassung an den Resonanzfall
ZL1:    Zuleitung Tip-Elektrode
ZL2:    Zuleitung Ring-Elektrode

## Patentansprüche

1.    Temporär oder permanent implantierbares medizinisches Gerät mit wenigstens einem ersten und einem zweiten langgestreckten elektrischen Funktionsleiter (ZL1, ZL2) für die Übertragung von Therapiesignalen oder Diagnosesignalen oder beidem, und einem mit einem der Funktionsleiter (ZL1, ZL2) verbundenen Elektrodenpol (22, 24, 30, 32, 210, 310, 410, 510, 610, 710, 220, 320, 420, 520, 720), über den elektrischer Strom an im Benutzungsfall an umgebendes Körpergewebe abgegeben oder mit dem elektrische Potentiale in im Benutzungsfall umgebenden Gewebe abgefühlt werden können oder beides,

wobei die beiden elektrischen Funktionsleiter (ZL1, ZL2) für definierte Resonanzfrequenzen derart induktiv gekoppelt sind, dass RF-Energie eines ersten Funktionsleiters (ZL1, ZL2) auf den zweiten Funktionsleiter (ZL1, ZL2) abgeleitet wird und die Energie über diesen Funktionsleiter (ZL1, ZL2) und einen mit diesem Funktionsleiter (ZL1, ZL2) verbundenen Elektrodenpol (22, 24, 30, 32, 210, 310, 410, 510, 610, 710, 220, 320, 420, 520, 720) an im Benutzungsfall umliegendes Gewebe abgegeben wird, **dadurch gekennzeichnet dass** das medizinische Gerät einen zwischen den ersten und den zweiten Funktionsleiter (ZL1, ZL2) geschalteten Transformator (230, 230', 530, 630, 730) zur induktiven Kopplung der beiden Funktionsleiter aufweist.

**2.** Medizinisches Gerät nach Anspruch 1, **dadurch gekennzeichnet, dass** das medizinische Gerät eine Stimulationselektrodenleitung (20) ist.

**3.** Medizinisches Gerät nach Anspruch 1 oder 2, **dadurch gekennzeichnet, dass** der zweite Funktionsleiter (ZL1, ZL2) mit wenigstens einem Elektrodenpol (22, 24, 30, 32, 210, 310, 410, 510, 610, 710, 220, 320, 420, 520, 720) in Form einer Ring (24) oder Coil-Elektrode (30, 32) verbunden ist.

**4.** Medizinisches Gerät nach einem der Ansprüche 1 bis 3, **dadurch gekennzeichnet, dass** das medizinische Gerät einen mit einer Wicklung des Transformators (230, 230', 530, 630, 730) parallel oder in Reihe geschalteten Kondensator (240, 460, 550, 650, 660, 750, 760) aufweist.

**5.** Medizinisches Gerät nach einem der Ansprüche 1 bis 4, **dadurch gekennzeichnet, dass** der Transformator (230, 230', 530, 630, 730) kernlos ist.

**6.** Medizinisches Gerät nach einem der Ansprüche 1 bis 5, **dadurch gekennzeichnet, dass** der Transformator (230, 230', 530, 630, 730) einen Kern aus ferromagnetischem Kernmaterial besitzt.

**7.** Medizinisches Gerät nach Anspruch 6, **dadurch gekennzeichnet, dass** der Transformator (230, 230', 530, 630, 730) einen Kern aus ferromagnetischem Kernmaterial besitzt, dessen Sättigung erst bei größerer als der zu erwartenden MRT-Magnetfeldstärke einsetzt

**8.** Medizinisches Gerät nach einem der Ansprüche 2 bis 7, **dadurch gekennzeichnet, dass** Transformator (230, 230', 530, 630, 730) und oder der Kondensator (240, 460, 550, 650, 660, 750, 760) auf eine Resonanzfrequenz abgestimmt ist, wobei eine im Benutzungsfall zu erwartende parasitäre Kapazität des Körpernetzwerkes bei der Abstimmung berücksichtigt ist.

**9.** Medizinisches Gerät nach einem der Ansprüche 1 bis 8, **dadurch gekennzeichnet, dass** der zweite Funktionsleiter (ZL1, ZL2) wenigstens mit einer zusätzlichen Ringelektrode (740) als speziell zur Ableitung induzierter RF-Energie vorgesehenem Elektrodenpol elektrisch verbunden ist.

**Claims**

**1.** A temporarily or permanently implantable medical device comprising at least one first and one second elongate electrical functional conductor (ZL1, ZL2) for transmitting therapeutic signals or diagnostic signals or both, and an electrode pole (22, 24, 30, 32, 210, 310, 410, 510, 610, 710, 220, 320, 420, 520, 720) which is connected to one of the functional conductors (ZL1, ZL2) and via which electrical current is delivered to surrounding bodily tissue during use, or with which electrical potentials in the surrounding tissue can be sensed during use, or both, wherein the two functional conductors (ZL1, ZL2) are inductively coupled for defined resonance frequencies, such that RF energy of a first functional conductor (ZL1, ZL2) is diverted to the second functional conductor (ZL1, ZL2), and the energy is delivered, via this functional conductor (ZL1, ZL2) and an electrode pole (22, 24, 30, 32, 210, 310, 410, 510, 610, 710, 220, 320, 420, 520, 720) connected to this functional conductor (ZL1, ZL2), to the surrounding tissue during use, **characterised in that** the medical device has a transformer (230, 230', 530, 630, 730) connected between the first and the second functional conductors (ZL1, ZL2) in order to inductively couple the two functional conductors.

**2.** The medical device according to Claim 1, **characterised in that** the medical device is a stimulation electrode lead (20).

**3.** The medical device according to Claim 1 or 2, **characterised in that** the second functional conductor (ZL1, ZL2) is connected to at least one electrode pole (22, 24, 30, 32, 210, 310, 410, 510, 610, 710, 220, 320, 420, 520, 720) in the form of a ring (24) or coil electrode (30, 32).

**4.** The medical device according to one of Claims 1 to 3, **characterised in that** the medical device comprises a capacitor (240, 460, 550, 650, 660, 750, 760) connected in parallel or in series with a winding of the transformer (230, 230', 530, 630, 730).

**5.** The medical device according to one of Claims 1 to 4, **characterised in that** the transformer (230, 230', 530, 630, 730) does not have a core.

**6.** The medical device according to one of Claims 1 to 5, **characterised in that** the transformer (230, 230', 530, 630, 730) has a core made of ferromagnetic core material.

7. The medical device according to Claim 6, **characterised in that** the transformer (230, 230', 530, 630, 730) has a core made of ferromagnetic core material, of which the saturation begins only at a magnetic field strength higher than the expected MRI magnetic field strength.

8. The medical device according to one of Claims 2 to 7, **characterised in that** the transformer (230, 230', 530, 630, 730) and/or the capacitor (240, 460, 550, 650, 660, 750, 760) is tuned to a resonance frequency, wherein a parasitic capacitance of the body network anticipated during use is taken into account in the tuning.

9. The medical device according to one of Claims 1 to 8, **characterised in that** the second functional conductor (ZL1, ZL2) is electrically connected at least to an additional ring electrode (740) as a special electrode pole intended to divert induced RF energy.

## Revendications

1. Appareil médical implantable de manière temporaire ou permanente avec au moins un premier et un deuxième conducteurs fonctionnels électriques (ZL1, ZL2) étirés en longueur, pour la transmission de signaux de thérapie ou de signaux de diagnostic, ou les deux, et un pôle d'électrode (22, 24, 30, 32, 210, 310, 410, 510, 610, 710, 220, 320, 420, 520, 720), relié avec un des conducteurs fonctionnels (ZL1, ZL2), par lequel le courant électrique est délivré, dans le cas de l'utilisation, au tissu corporel environnant ou, dans le cas de l'utilisation, les potentiels électriques dans les tissus environnants peuvent être détectés, ou les deux, où les deux conducteurs fonctionnels électriques (ZL1, ZL2) sont couplés de manière inductive pour des fréquences de résonance définies de telle sorte que l'énergie de radiofréquence RF d'un premier conducteur fonctionnel (ZL1, ZL2) est dérivée sur le deuxième conducteur fonctionnel (ZL1, ZL2), et l'énergie est délivrée dans le tissu environnant en cas d'utilisation par l'intermédiaire de ces conducteurs fonctionnels (ZL1, ZL2) et de l'un des pôles d'électrode (22, 24, 30, 32, 210, 310, 410, 510, 610, 710, 220, 320, 420, 520, 720) relié avec ce conducteur fonctionnel (ZL1, ZL2), **caractérisé en ce que** l'appareil médical présente un transformateur (230, 230', 530, 630, 730) branché entre les premier et deuxième conducteurs fonctionnels (ZL1, ZL2) pour le couplage inductif des deux conducteurs fonctionnels.

2. Appareil médical selon la revendication 1, **caractérisé en ce que** l'appareil médical est un fil d'électrode de stimulation (20).

3. Appareil médical selon les revendications 1 ou 2, **caractérisé en ce que** le deuxième conducteur fonctionnel (ZL1, ZL2) est relié avec au moins un pôle d'électrode (22, 24, 30, 32, 210, 310, 410, 510, 610, 710, 220, 320, 420, 520, 720) sous la forme (24) ou d'une électrode de bobine (30, 32) d'une électrode annulaire.

4. Appareil médical selon l'une des revendications 1 à 3, **caractérisé en ce que** l'appareil médical présente un condensateur (240, 460, 550, 650, 660, 750, 760) branché en parallèle ou en série avec une bobinage du transformateur (230, 230', 530, 630, 730.

5. Appareil médical selon l'une des revendications 1 à 4, **caractérisé en ce que** le transformateur (230, 230', 530, 630, 730) est sans noyau.

6. Appareil médical selon l'une des revendications 1 à 5, **caractérisé en ce que** le transformateur (230, 230', 530, 630, 730) possède un noyau en une matière de coeur ferromagnétique.

7. Appareil médical selon la revendication 6, **caractérisé en ce que** le transformateur (230, 230', 530, 630, 730) possède un noyau en une matière de coeur ferromagnétique dont la saturation ne se produit que pour des puissances de champ magnétique d'IRM supérieures à celles attendues.

8. Appareil médical selon l'une des revendications 2 à 7, **caractérisé en ce que** le transformateur (230, 230', 530, 630, 730) et ou le condensateur (240, 460, 550, 650, 660, 750, 760) sont réglés sur une fréquence de résonance, où, dans le cas de l'utilisation, il est tenu compte d'une capacité parasite du réseau corporel attendue lors de l'évaluation.

9. Appareil médical selon l'une des revendications 1 à 8, **caractérisé en ce que** le deuxième conducteur fonctionnel (ZL1, ZL2) est relié électriquement au moins avec une électrode annulaire (740) supplémentaire spécialement prévue en tant que pôle d'électrode pour la décharge de l'énergie RF induite.

FIG. 1

FIG. 2

FIG. 3A

FIG. 3B

FIG. 4

FIG. 5

EP 2 465 570 B1

FIG. 6

FIG. 7

FIG. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20100016936 A1